# EUROPEAN PATENT APPLICATION

(11) **EP 4 108 157 A1**
(43) Date of publication of application: **28.12.2022**
(21) Application number: 22180555.9
(22) Date of filing: 22.06.2022
(51) Int. Cl.: A61B 1/00, A61B 5/06, A61B 34/20

(54) **MEDICAL IMAGE PROCESSING DEVICE, OPERATION METHOD THEREFOR, AND ENDOSCOPE SYSTEM**

(30) Priority: 23.06.2021 JP 2021104333
(71) Applicant: FUJIFILM Corporation, Tokyo 106-8620 (JP)
(72) Inventor: OYATSU, Masayuki, Kanagawa (JP)
(74) Representative: Jeffrey, Philip Michael

(57) **Abstract**

Disclosed herein is a medical image processing device (17), an operation method therefor, and an endoscope system (10) capable of displaying a gravity direction without installing a special device in an endoscope (12). A medical image processing device (17) disclosed herein acquires an endoscopic image obtained by imaging a subject with an endoscope (12), detects a gravity direction in the endoscopic image on the basis of the endoscopic image, generates a gravity direction indication indicating the gravity direction, and displays the gravity direction indication on a display (15). An endoscope system (10) disclosed herein includes the medical image processing device (17) and the endoscope (12).

## Description

### BACKGROUND OF THE INVENTION

### 1. Field of the Invention

The present invention relates to a medical image processing device, an operating method therefor, and an endoscope system.

### 2. Description of the Related Art

In recent years, diagnosis or treatment using an endoscope has been actively performed in the medical field. For example, endoscopic submucosal dissection (ESD) makes it possible to resect tumors or the like with a size to which endoscopic mucosal resection (EMR) cannot be applied and thus to complete an operation without selecting a highly invasive surgery. ESD is performed endoscopically, and thus has the advantage of being minimally invasive. On the other hand, a doctor who is an operator is required to perform ESD with limited information based on an image (hereinafter referred to as an endoscopic image) obtained by imaging an observation target of an endoscope, which is a subject, with the endoscope, and is thus required to have a practiced skill.

There are a system and a method for displaying an indicator that gives a vertical orientation of an endoscopic image with a rotation sensor or the like attached to a housing or the like in order for a doctor to distinguish between the top and the bottom in the endoscopic image (JP2007-275257A).

### SUMMARY OF THE INVENTION

In a case where a gravity direction is detected by a physical method such as a rotation sensor, it is difficult to use an endoscope as usual, and it may be necessary to install a special device in the endoscope. Installing a special device leads to an increase in the size of the endoscope.

The present invention provides a medical image processing device, an operation method therefor, and an endoscope system capable of displaying a gravity direction without installing a special device in an endoscope.

According to an aspect of the present invention, there is provided an endoscopic image processing device including a processor, in which the processor acquires a medical image obtained by imaging a subject with an endoscope, detects a gravity direction in the endoscopic image on the basis of the endoscopic image, generates a gravity direction indication indicating the gravity direction, and performs control for displaying the gravity direction indication on a display.

The subject preferably includes a liquid accumulation.

The subject preferably includes a mucous membrane of which at least a part is incised.

The subject preferably includes a released liquid.

The processor preferably detects the gravity direction in the endoscopic image by using the endoscopic image and a learning model that outputs the gravity direction in the endoscopic image in a case where the endoscopic image is input.

It is preferable that the processor generates a superimposition image in which the gravity direction indication is superimposed on the medical image, and performs control for displaying the superimposition image on the display.

The processor preferably performs control for displaying the gravity direction indication with at least one of a color, a symbol, or a figure.

The processor preferably performs control for displaying the gravity direction indication with a color around the endoscopic image.

The processor preferably controls whether or not to display the gravity direction indication on the basis of a user's instruction.

The processor preferably controls whether or not to display the gravity direction indication on the basis of the endoscopic image.

The processor preferably determines a display mode of the gravity direction indication on the basis of the endoscopic image.

According to another aspect of the present invention, there is provided an operation method for an endoscopic image processing device, including a step of acquiring a medical image obtained by imaging a subject with an endoscope; a step of detecting a gravity direction in the endoscopic image on the basis of the endoscopic image; a step of generating a gravity direction indication indicating the gravity direction; and a step of performing control for displaying the gravity direction indication on a display.

According to still aspect of the present invention, there is provided an endoscope system including the medical image processing device and the endoscope.

According to the present invention, a gravity direction can be displayed without installing a special device in an endoscope.

### BRIEF DESCRIPTION OF THE DRAWINGS

Fig. 1 is a schematic diagram of an endoscope system.
Fig. 2 is a block diagram showing a function of the endoscope system.
Fig. 3 is an explanatory diagram for describing four-color LEDs included in a light source unit.
Fig. 4 is a graph showing spectra of violet light V, blue light B, green light G, and red light R.
Fig. 5 is a block diagram showing a function of a medical image processing device.
Fig. 6 is an explanatory diagram for describing a function of a direction detection unit.
Fig. 7 is an image diagram showing an example of a normal image used for a learning image.
Fig. 8 is an explanatory diagram for describing an example of a method of creating a learning image.
Fig. 9 is an explanatory diagram for describing an example of a method of creating a learning image.
Fig. 10 is an explanatory diagram for describing an example of details output by the direction detection unit.
Fig. 11 is an explanatory diagram for describing an example of details output by the direction detection unit.
Fig. 12 is an image diagram showing an example of a gravity direction indication.
Fig. 13 is an image diagram showing an example of a gravity direction indication.
Fig. 14 is an image diagram showing an example of a superimposition image.
Fig. 15 is an image diagram showing an example of a superimposition image.
Fig. 16 is an image diagram of a normal image including a liquid accumulation in a subj ect.
Fig. 17 is an image diagram of a normal image including an incised mucous membrane in the subject.
Fig. 18 is an image diagram of a normal image including a released liquid in the subj ect.
Fig. 19 is a block diagram showing a function of the medical image processing device including an image analysis unit.
Fig. 20 is an explanatory diagram for describing that a gravity direction indication is not automatically displayed.
Fig. 21 is an explanatory diagram for describing that a gravity direction indication is automatically displayed.
Fig. 22 is a block diagram showing a function of the medical image processing device including a posture information unit.
Fig. 23 is an explanatory diagram for describing posture information.
Fig. 24 is an explanatory diagram for describing a case where the medical image processing device is included in a diagnosis support device.
Fig. 25 is an explanatory diagram for describing a case where the medical image processing device is included in a medical service support device.

### DESCRIPTION OF THE PREFERRED EMBODIMENTS

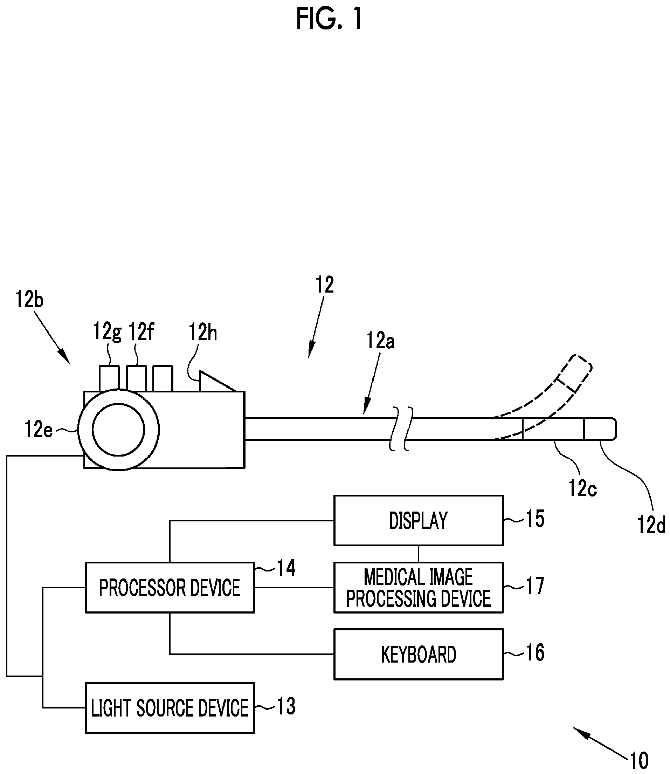

As shown in Fig. 1, an endoscope system 10 includes an endoscope 12, a light source device 13, a processor device 14, a display 15, a keyboard 16, and a medical image processing device 17. The endoscope 12 is optically connected to the light source device 13 and electrically connected to the processor device 14. The processor device 14 is connected to the medical image processing device 17. The medical image processing device 17 acquires an endoscopic image that is a medical image from the processor device 14, and performs various processes for acquiring various types of information and the like.

In the present embodiment, the medical image is an endoscopic image. In the present embodiment, the medical image processing device 17 and the processor device 14 are separate devices, but a device that functions as the medical image processing device 17 may be disposed in the processor device 14, and the processor device 14 may perform the function of the medical image processing device 17. The various connections are not limited to wired connections, and may be wireless connections, or may be connections using a network. Therefore, the function of the medical image processing device 17 may be performed by an external device connected thereto via a network.

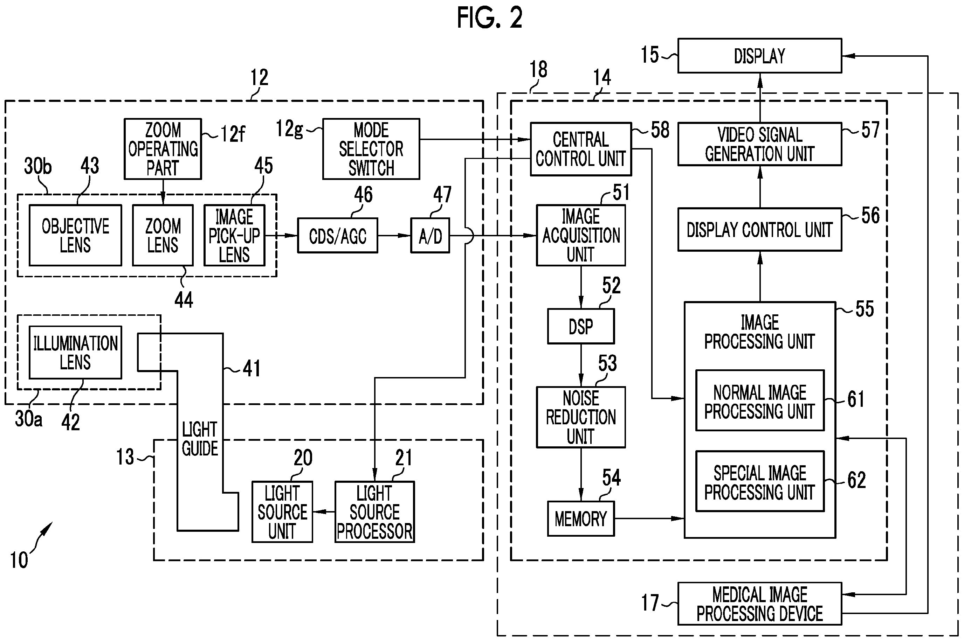

The endoscope 12 is provided with an insertion part 12a to be inserted into the body of a subject having an observation target, an operating part 12b provided at a base end portion of the insertion part 12a, and a bendable part 12c and a tip part 12d provided at a distal end side of the insertion part 12a. The bendable part 12c is curved by operating an angle knob 12e (refer to Fig. 2) of the operating part 12b. The tip part 12d is directed in a desired direction when the bendable part 12c is curved. A forceps channel (not shown) for inserting a treatment tool or the like is provided from the insertion part 12a to the tip part 12d. The treatment tool is inserted into the forceps channel from a forceps port 12h. Air supply, water supply, or suction is also performed from the forceps port 12h.

The operating part 12b includes a zoom operating part 12f for changing an image pick-up magnification and a mode selector switch 12g used for an observation mode switching operation, in addition to the angle knob 12e. An observation mode switching operation or a zoom operation may be an operation or an instruction using a keyboard 16 or a foot switch (not shown) in addition to the mode selector switch 12g or the zoom operating part 12f.

The endoscope system 10 has a normal observation mode in which a normal image, which is an image of a natural color obtained by picking up an image of an observation target by using white light as illumination light, is displayed on the display 15. In addition to the normal observation mode, a special observation mode in which a special image that is an endoscopic image obtained by emitting illumination light having a specific spectrum different from that of white light to acquire a special image of an observation target is displayed on the display 15, and a multi-observation mode that automatically switches between the normal observation mode and the special observation mode may be provided. In the present embodiment, an endoscopic image is acquired in the normal observation mode. As the special image, an endoscopic image emphasizing a specific structure such as a blood vessel or a duct can be displayed on the display 15, and therefore, in some cases, a mode other than the normal observation mode is used.

The processor device 14 is electrically connected to the medical image processing device 17, the display 15, and the keyboard 16. The display 15 displays, for example, a normal image, a special image, and/or various types of information. The keyboard 16 functions as a user interface that receives input operations such as function settings. An external storage (not shown) for storing images, image information, and the like may be connected to the processor device 14.

As shown in Fig. 2, the light source device 13 includes a light source unit 20 that emits illumination light to irradiate an observation target, and a light source processor 21 that controls the light source unit 20. The light source unit 20 is configured with, for example, a semiconductor light source such as multi-color light emitting diodes (LEDs), a combination of a laser diode and a phosphor, or a xenon lamp or a halogen light source. The light source unit 20 includes an optical filter or the like for adjusting a wavelength range of light emitted by the LED or the like. The light source processor 21 controls an amount of illumination light by turning on/off each LED and the like and adjusting a drive current or a drive voltage of each LED and the like. The light source processor 21 controls a wavelength range of the illumination light by changing an optical filter or the like.

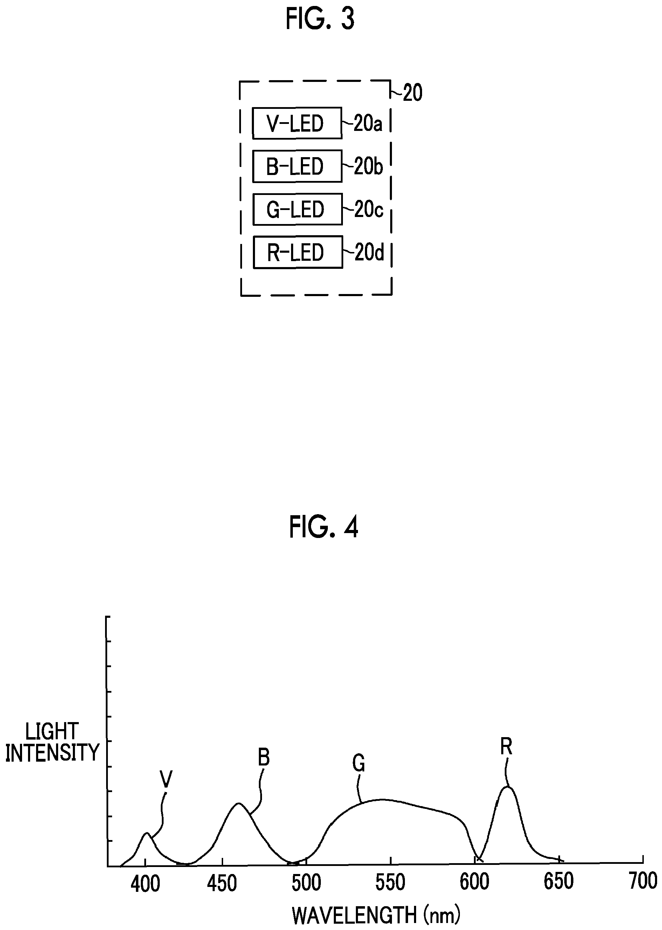

As shown in Fig. 3, in the present embodiment, the light source unit 20 includes four color LEDs such as a violet light emitting diode (V-LED) 20a, a blue light emitting diode (B-LED) 20b, and a green light emitting diode (G-LED) 20c, and a red light emitting diode (R-LED) 20d.

As shown in Fig. 4, the V-LED 20a generates violet light V having a central wavelength of 410±10 nm and a wavelength range of 380 to 420 nm. The B-LED 20b generates blue light B having a central wavelength of 450±10 nm and a wavelength range of 420 to 500 nm. The G-LED 20c generates green light G having a wavelength range of 480 to 600 nm. The R-LED 20d generates red light R having a central wavelength of 620 to 630 nm and a wavelength range of 600 to 650 nm.

The light source processor 21 controls the V-LED 20a, the B-LED 20b, the G-LED 20c, and the R-LED 20d. In the normal observation mode, the light source processor 21 controls the respective LEDs 20a to 20d such that normal light in which a combination of the light intensity ratios between the violet light V, the blue light B, the green light G, and the red light R is Vc:Bc:Gc:Rc is emitted.

in a case where the special observation mode is set, the light source processor 21 changes, for example, a combination of the light intensity ratios between the violet light V, the blue light B, the green light G, and the red light R such that illumination light having a specific spectrum is emitted.

The light emitted by each of the LEDs 20a to 20e is incident to a light guide 41 via an optical path coupling portion (not shown) configured with a mirror, a lens, and the like. The light guide 41 is built in the endoscope 12 and a universal cord (a cord connecting the endoscope 12, the light source device 13, and the processor device 14 to each other). The light guide 41 propagates light from the optical path coupling portion to the tip part 12d of the endoscope 12.

An illumination optical system 30a and an image pick-up optical system 30b are provided at the tip part 12d of the endoscope 12. The illumination optical system 30a has an illumination lens 42, and the illumination light propagated by the light guide 41 is applied to the observation target via the illumination lens 42. The image pick-up optical system 30b has an objective lens 43, a zoom lens 44, and an image pick-up sensor 45. Various types of light such as reflected light, scattered light, and fluorescence from the observation target are incident to the image pick-up sensor 45 via the objective lens 43 and the zoom lens 44. Consequently, an image of the observation target is formed on the image pick-up sensor 45. The zoom lens 44 freely moves between the telephoto end and the wide end by operating the zoom operating part 12f, and enlarges or reduces the image of the observation target formed on the image pick-up sensor 45.

The image pick-up sensor 45 is a color image pick-up sensor provided with one of a red (R), green (G), or blue (B) color filter for each pixel, and picks up an image of the observation target to output an image signal of each color of RGB. As the image pick-up sensor 45, a charge coupled device (CCD) image pick-up sensor or a complementary metal oxide semiconductor (CMOS) image pick-up sensor may be used. Instead of the image pick-up sensor 45 provided with the primary color filter, a complementary image pick-up sensor provided with cyan (C), magenta (M), yellow (Y), and G (green) complementary filters may be used. In a case where a complementary image pick-up sensor is used, image signals of four colors of CMYG are output. Therefore, the same RGB image signals as in the image pick-up sensor 45 can be obtained by converting image signals of the four colors of CMYG into image signals of the three colors of RGB through complementary-primary color conversion. Instead of the image pick-up sensor 45, a monochrome sensor without a color filter may be used.

The image pick-up sensor 45 is driven and controlled by an image pick-up control unit (not shown). A central control unit 58 (refer to Fig. 2) controls light emission of the light source unit 20 via the light source processor 21 in synchronization with the image pick-up control unit, and thus in the normal observation mode, an image of an observation target illuminated by the normal light is picked up. Consequently, a Bc image signal is output from a B pixel of the image pick-up sensor 45, a Gc image signal is output from a G pixel, and an Rc image signal is output from an R pixel.

A correlated double sampling/automatic gain control (CDS/AGC) circuit 46 performs correlated double sampling (CDS) or automatic gain control (AGC) on an analog image signal obtained from the image pick-up sensor 45. The image signal that has passed through the CDS/AGC circuit 46 is converted into a digital image signal by an analog/digital (A/D) converter 47. The digital image signal after A/D conversion is input to the processor device 14.

In the processor device 14, a program related to processes such as image processing is stored in a program memory (not shown). In the processor device 14, the program in the program memory is operated by the central control unit 58 configured with an image processor or the like that is a first processor, to realize functions of an image acquisition unit 51, a digital signal processor (DSP) 52, a noise reduction unit 53, a memory 54, an image processing unit 55, a display control unit 56, a video signal generation unit 57, and the central control unit 58. The central control unit 58 receives information from the endoscope 12 and the light source device 13, and controls each unit of the processor device 14 and also controls the endoscope 12 or the light source device 13 on the basis of the received information. The central control unit 58 also receives information such as instructions from the keyboard 16.

The image acquisition unit 51 acquires a digital image signal for an endoscopic image input from the endoscope 12. The image acquisition unit 51 acquires an image signal obtained by imaging an observation target illuminated by each type of illumination light for each frame. The image acquisition unit 51 may acquire an endoscopic image obtained by imaging an observation target illuminated by types of illumination light having predetermined and different spectra.

The acquired image signal is transmitted to the DSP 52. The DSP 52 performs digital signal processing such as a color correction process on the received image signal. The noise reduction unit 53 performs a noise reduction process based on, for example, a moving average method, or a median filter method on the image signal subjected to the color correction process or the like by the DSP 52. The image signal with reduced noise is stored in the memory 54.

The image processing unit 55 acquires an image signal after noise reduction from the memory 54. The acquired image signal is subjected to signal processing such as a color conversion process, a color enhancement process, and a structure enhancement process as necessary to generate a color endoscopic image in which the observation target is captured. The image processing unit 55 includes a normal image processing unit 61 and a special image processing unit 62.

In the image processing unit 55, the normal image processing unit 61 performs image processing for a normal image such as a color conversion process, a color enhancement process, and a structure enhancement process on the input image signal for the normal image after noise reduction for one frame in the normal observation mode or the multi-observation mode. The image signal subjected to the image processing for a normal image is input to the medical image processing device 17 and/or the display control unit 56.

In the special observation mode or the multi-observation mode, the special image processing unit 62 performs image processing for the first image such as a color conversion process, a color enhancement process, and a structure enhancement process on the input image signal for the first image after noise reduction for one frame. The image signal subjected to image processing for the first image is input to the medical image processing device 17 and/or the display control unit 56 as the first image. The image processing unit 55 may adjust a frame rate in a case where the endoscopic image is input to the medical image processing device 17 and/or the display control unit 56.

The endoscopic image generated by the image processing unit 55 is a normal image in a case where an observation mode is the normal observation mode, and is a special image in a case where an observation mode is the special observation mode, and details of the color conversion process, the color enhancement process, and the structure enhancement process differ depending on the observation modes. In the normal observation mode, the image processing unit 55 generates a normal image by performing the various types of signal processing described above such that the observation target has a natural hue. In the special observation mode, the image processing unit 55 generates a special image by, for example, performing the various types of signal processing for emphasizing a blood vessel of the observation target.

The display control unit 56 receives the endoscopic image generated by the image processing unit 55 and performs control for displaying the endoscopic image on the display 15. The endoscopic image controlled to be displayed by the display control unit 56 is generated as a video signal to be displayed on the display 15 by the video signal generation unit 57, and is sent to the display 15. The display 15 displays the endoscopic image sent from the video signal generation unit 57 under the control of the display control unit 56.

The medical image processing device 17 acquires the endoscopic image generated by the image processing unit 55, detects a gravity direction in the endoscopic image on the basis of the endoscopic image, generates a gravity direction indication indicating the gravity direction, and performs control for displaying the gravity direction indication on the display.

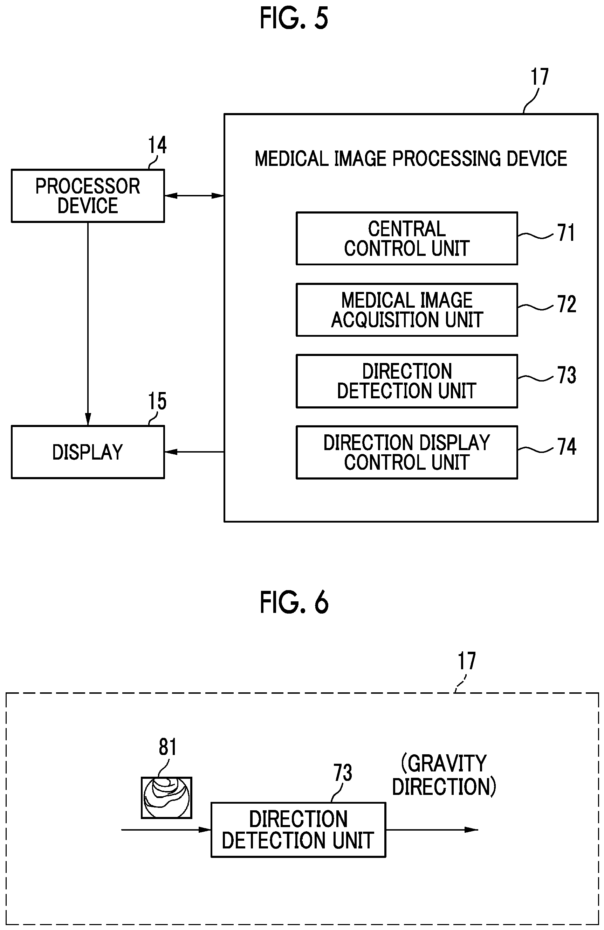

The medical image processing device 17 is a general-purpose PC provided with a processor, and exhibits various functions with installation of software. In the same manner as in the processor device 14, the medical image processing device 17 also stores a program related to processes such as an image analysis process in a program memory (not shown). In the medical image processing device 17, a central control unit 71 (refer to Fig. 5) configured with an image processor or the like that is a second processor operates the program in the program memory to realize functions of a medical image acquisition unit 72, a direction detection unit 73, and a direction display control unit 74 (refer to Fig. 5).

The medical image processing device 17 is connected to the display 15, and the display 15 displays various types of information generated and output by the medical image processing device 17. Various devices may be connected to the medical image processing device 17. Examples of various devices include a user interface such as a keyboard for giving instructions and the like, and a storage that stores data such as images and information. The medical image processing device 17 has a network connection function for connecting to various devices. The medical image processing device 17 may be connected to, for example, a medical service support device 630 (refer to Fig. 26) by the network connection function.

As shown in Fig. 5, the medical image processing device 17 includes the central control unit 71, the medical image acquisition unit 72, the direction detection unit 73, and the direction display control unit 74. The central control unit 71 controls each unit of the medical image processing device 17, receives information from the processor device 14 and the like, and controls each unit of the medical image processing device 17 on the basis of the received information. The central control unit 71 is also connected to a user interface such as a keyboard (not shown) and receives information such as instructions from the user interface.

The medical image acquisition unit 72 acquires a plurality of types of endoscopic images sent from the processor device 14. The acquired image is sent to the direction detection unit 73. The direction detection unit 73 detects a gravity direction in the endoscopic image on the basis of the endoscopic image acquired by the medical image acquisition unit 72. The gravity direction is a direction of gravity, and a destination to which gravity is directed is the bottom and a direction opposite to the bottom is the top. The direction display control unit 74 generates a gravity direction indication for providing a notification of the gravity direction detected by the direction detection unit 73, and performs control of displaying the gravity direction indication on the display 15.

In the present embodiment, the medical image acquisition unit 72 acquires a normal image obtained by imaging an observation target illuminated by normal light with the endoscope from the processor device 14, and sends the normal image to the direction detection unit 73. The direction detection unit 73 detects a gravity direction in the normal image sent from the medical image acquisition unit 72. In some cases, the medical image acquisition unit 72 may acquire an image other than the normal image, such as a special image, and the direction detection unit 73 may detect a gravity direction in the image other than the normal image, such as a special image, acquired by the medical image acquisition unit 72. The special image includes, for example, an image in which a specific structure such as a superficial blood vessel is emphasized, and may include information that cannot be ascertained from a normal image. Therefore, in some cases, by detecting a gravity direction in the special image, it may be possible to detect the gravity direction more accurately and precisely.

Generally, in a case where a user such as a doctor ascertains a gravity direction in an endoscopic image, the gravity direction is ascertained on the basis of a patient's posture and a landmark captured in the endoscopic image. The landmark is a structure that is characteristic of a specific site of an organ, and the landmark makes it possible to ascertain an imaging position in the lumen including the gravity direction. However, for example, for detailed observation of an observation target, the landmark may not be captured in the endoscope such as a case where the endoscope is in close proximity to the observation target. In a case where the landmark changes frequently due to the progress of endoscopy, a user has to ascertain a gravity direction at that time while following the changing gravity direction, which is troublesome. Therefore, by indicating the gravity direction in such a case, the user can concentrate on work other than ascertaining the gravity direction.

The direction detection unit 73 detects a gravity direction in a normal image on the basis of the normal image. As a detection method, image processing or machine learning techniques may be used. It is preferable to use a learning model in machine learning because there is a possibility that a gravity direction can be detected accurately and more reliably. As the learning model, a model generated in advance by performing learning and adjustment such that an accurate gravity direction is output in a case where a normal image is input is used.

As shown in Fig. 6, the direction detection unit 73 is a learning model that detects and outputs a gravity direction in a normal image 81 in a case where the normal image 81 is input, and is specifically a program. The learning model can employ various techniques in machine learning and can make various adjustments in order to detect a position of a specific blood vessel with higher accuracy. Since the position of the specific blood vessel may be obtained with higher accuracy, the direction detection unit 73 is preferably a multi-layer neural network model. A learning model detects a gravity direction by receiving an image such as the normal image 81, and may thus be a convolutional neural network model or a deep learning model.

The direction detection unit 73 is generated in advance through learning using a learning image. The learning image is an endoscopic image associated with a direction of gravity in the normal image 81. The learning image is preferably a learning image including a scene in which gravity is desired to be detected, and the learning model is preferably a model having performed the learning image including a scene in which gravity is desired to be detected. For example, specifically, in a case where the direction detection unit 73 of the present embodiment detects and displays a gravity direction in the endoscopy of the upper gastrointestinal tract, it is preferable to use a learning model having performed a learning image in which the gravity direction is associated with the normal image 81 of the upper gastrointestinal tract.

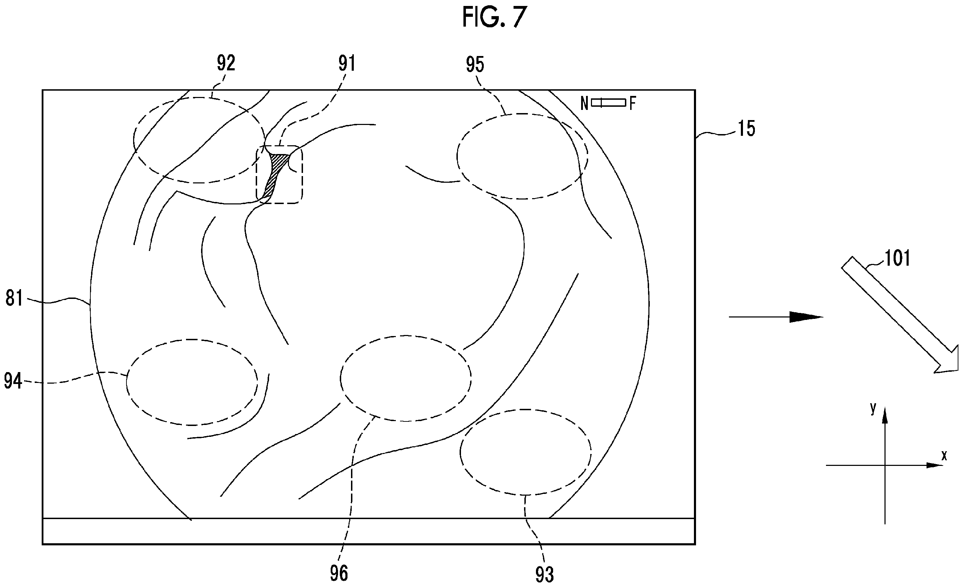

As shown in Fig. 7, specifically, as the learning image, the normal image 81 in which the stomach is observed as the upper gastrointestinal tract may be used. In the present embodiment, the normal image 81 displayed on the display 15 is a normal image 81 in which a pyloric portion 91 of the stomach is included in an observation target. In addition to the pyloric portion 91, a lesser curvature 92, a greater curvature 93, an anterior wall 94, a posterior wall 95, and a vestibular portion 96 are included in the observation target, and thus these sites can be used as landmarks for obtaining a gravity direction. With these sites as landmarks, a gravity direction in the normal image 81 is a lower right direction as indicated by a gravity direction 101 on a plane formed by the xy axes. In a case where a gravity direction in the normal image 81 is directly above or directly below and cannot be indicated by an arrow or the like, the gravity direction may be indicated by being displayed with text such as "directly above" or "directly below".

Actually, the gravity direction 101 is a three-dimensional vector in a three-dimensional space including the observation target. However, the gravity direction 101 is a gravity direction in the plane formed by the xy axes perpendicular to the optical axis of the image pick-up optical system 30b of the endoscope. It can be said that, in the normal image 81, the observation target and the tip part 12d of the endoscope face each other in parallel, and, in relation to a direction indicated by the gravity direction 101, in a case where a staining solution is applied to the observation target, the staining solution flows in the gravity direction 101. Since a direction in which a liquid flows can be roughly recognized, the associated gravity direction 101 may be a two-dimensional direction in a plane perpendicular to the optical axis of the image pick-up optical system 30b of the endoscope as shown in Fig. 7. More accurately, the gravity direction 101 may be a three-dimensional direction in a three-dimensional space including a subject captured in the normal image 81.

Associating is to associate the normal image 81 with the gravity direction 101 in the normal image 81. Both the normal image 81 and the gravity direction 101 may be associated with each other, and in a case where the normal image 81 is selected, the gravity direction 101 in the normal image 81 may be recognized. Any associating method may be used. So-called tagging may be performed on the normal image 81. Image data of the normal image 81 may include information regarding the gravity direction 101 as a header or the like, or a table-like format in which the normal image 81 and the information regarding the gravity direction 101 are stored may be used. In Fig. 7, the normal image 81 and the gravity direction 101 are associated by causing the vector of the gravity direction 101 to be included in the data of the normal image 81.

The gravity direction 101 associated with the normal image 81 is preferably accurate as much as possible, but it does not have to be highly accurate, and may be obtained by a skilled doctor by visually observing the normal image 81, or may be obtained through image analysis of the normal image 81 or through machine learning or the like based on the normal image 81. In a case where it is only necessary to know a direction or a position where a liquid is collected or a direction in which an incised mucous membrane hangs down, the gravity direction 101 associated with the normal image 81 may have an error of plus or minus 45 degrees or less with the actual gravity direction, that is, about 90 degrees or less in total. This is because the direction in which the liquid is collected or the direction in which the incised mucous membrane hangs down can be roughly ascertained.

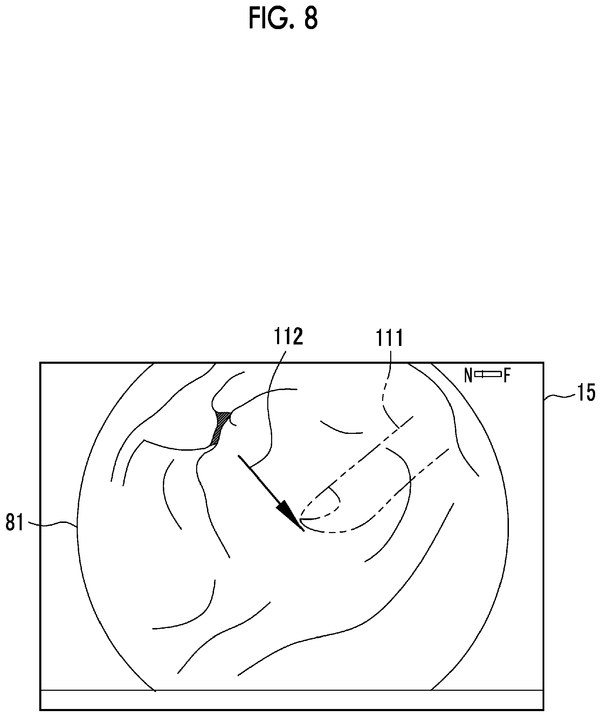

As a method for a doctor to obtain the gravity direction 101 in the normal image 81 and associate the gravity direction 101 with the normal image 81, for example, there may be a method in which the normal image 81 is displayed on the display 15, and the gravity direction 101 is designated by visually observing the normal image 81. As a method of designating the gravity direction 101, for example, as a method of designating the gravity direction in two dimensions, as shown in Fig. 8, there may be a method in which, on the display 15, a downward direction is simply written by an arrow 112 with a mouse, a finger 111, or the like on the normal image 81.

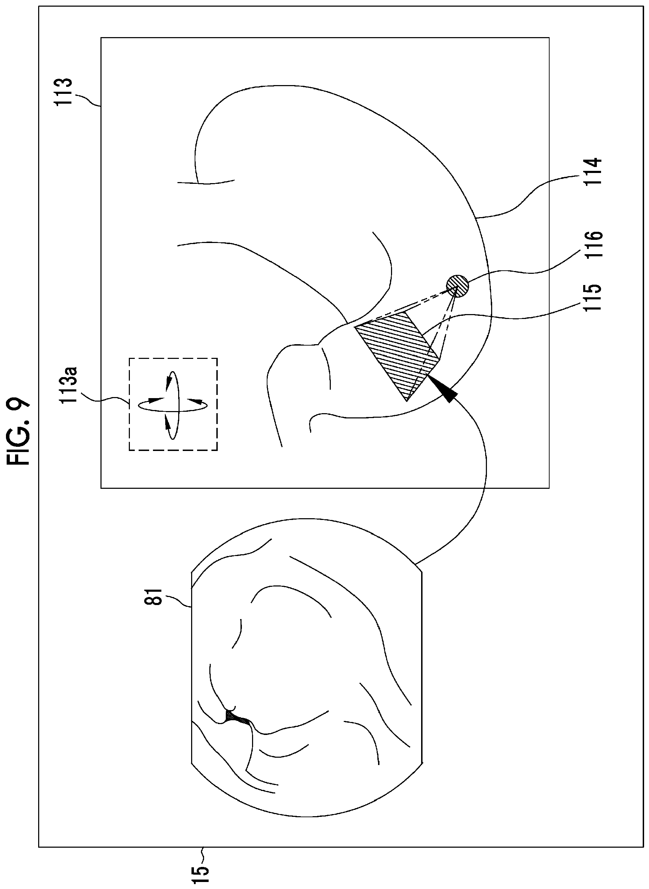

Regarding designation of the gravity direction 101 in three dimensions, as shown in Fig. 9, for example, there may be a method of designating the gravity direction 101 by using a gravity direction designation screen 113. The gravity direction designation screen 113 includes the normal image 81 and a gravity direction designation screen 113, and the gravity direction designation screen 113 includes a three-dimensional schema diagram 114 of each site or the like. A rotatable indication 113a indicates that the three-dimensional schema diagram 114 is rotatable. In the present embodiment, the three-dimensional schema diagram 114 of the stomach is used. A camera position 116 may also be included.

The gravity direction 101 is designated, specifically, by displaying the normal image 81 and the gravity direction designation screen 113 on the display 15, and attaching the normal image 81 to an imaging position 115 of the three-dimensional schema diagram 114 on the display 15. The three-dimensional schema diagram 114 of each site and the camera position 116 can be moved three-dimensionally. A doctor or the like visually observes the normal image 81 and drags the position of the normal image 81 to the position of the normal image 81 in the three-dimensional schema diagram114 on the display 15. In a case where the position of the normal image 81 in the three-dimensional schema diagram114 is determined, the imaging position 115 is determined, and for example, a display color of the imaging position 115 on the display 15 changes. The icon of the camera position 116 can also be moved.

Information regarding a patient's posture having the observation target of which the normal image 81 is captured is acquired. The posture information of the patient is used to determine a position where the three-dimensional schema diagram 114 is displayed on the display. In the above-described way, the gravity direction 101 can be obtained by designating the imaging position 115. The three-dimensional schema diagram 114 can be freely moved, and the accurate gravity direction 101 in the normal image 81 may be obtained by being calculated from the posture information of a patient and the normal image 81 attached to the three-dimensional schema diagram 114 of the stomach. In addition to these methods, various methods may be used as long as the gravity direction 101 in the normal image 81 can be associated according to the methods.

In a case where a gravity direction in a normal image is obtained through image analysis or machine learning and associated with the normal image, for example, the gravity direction 101 may be obtained as a vector in three-dimensional coordinates and associated with the normal image.

A method of writing a direction in two dimensions into the normal image 81 is preferable because a doctor or the like can easily designate the gravity direction 101 in the normal image 81, and for example, in a case where a cleaning liquid is released or bleeding occurs due to an endoscope, a direction in which the liquid flows can be detected. A method using a three-dimensional model or a three-dimensional vector is preferable because the gravity direction 101 can be indicated even in a case where the normal image 81 is captured in a direct upward direction or a direct downward direction.

The direction detection unit 73 includes a learning model. The direction detection unit 73 detects the gravity direction 101 on the basis of the normal image 81 by using the learning model. The learning model performs learning by using the learning image as described above. Thereafter, a test is performed by using a test image associated with the gravity direction 101, and a learning model is generated by adjusting parameters such that the accurate gravity direction 101 associated with the test image is output. Since the generated learning model is a learning model in which the gravity direction 101 is output in a case where the normal image 81 is input, the learning model detects and outputs the gravity direction 101 in the input normal image 81 in a case where the normal image 81 in which the gravity direction 101 is desired to be known is input. The output gravity direction 101 is sent to the direction display control unit 74.

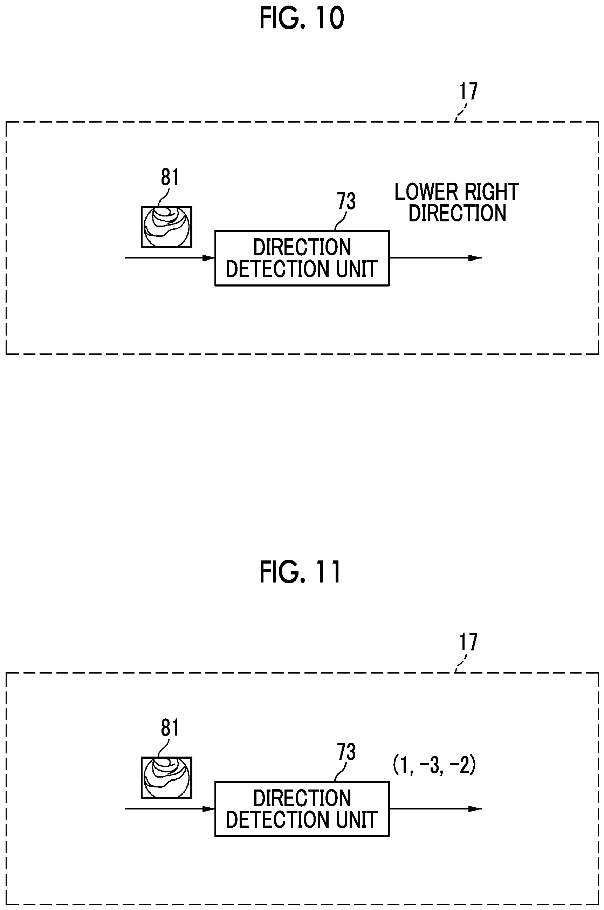

As shown in Fig. 10, in a case where the normal image 81 that is a detection target is input to the direction detection unit 73 having the learning model, the learning model that detects the gravity direction 101 in two dimensions detects the gravity direction 101 in the normal image 81 and outputs a "lower right direction". As shown in Fig. 11, the learning model that detects the gravity direction 101 in three dimensions detects the gravity direction in the normal image 81 and outputs the gravity direction in a three-dimensional vector as in "(1, -3, -2)".

The detected gravity direction 101 is sent to the direction display control unit 74. In order to notify a user of the gravity direction 101, the direction display control unit 74 displays a gravity direction indication in a method of allowing the user to recognize the gravity direction. The gravity direction indication may be an image indicating the gravity direction 101 in the normal image 81.

Details of the gravity direction indication may be set in advance by a user. For example, a frequency of the gravity direction indication may be set such that the gravity direction indication is not updated in a case where a change in the gravity direction 101 is within a predetermined angle in order to prevent the gravity direction indication from changing frequently. The predetermined angle is set such that, for example, in a case where a difference between angles before and after the change is plus or minus 45 degrees or less, that is, 90 degrees or less in total, the indication is not updated, and in a case where the difference is 90 degrees or more, the indication is updated.

For example, the gravity direction indication may be superimposed on the normal image 81 and displayed. In this case, a user may set a position of the gravity direction indication on the normal image 81 in advance. For example, the gravity direction indication is set to be displayed at a position that does not interfere with the user's observation, or the gravity direction indication is set to be displayed in an inconspicuous display mode such as transparency and displayed at the center of the normal image 81.

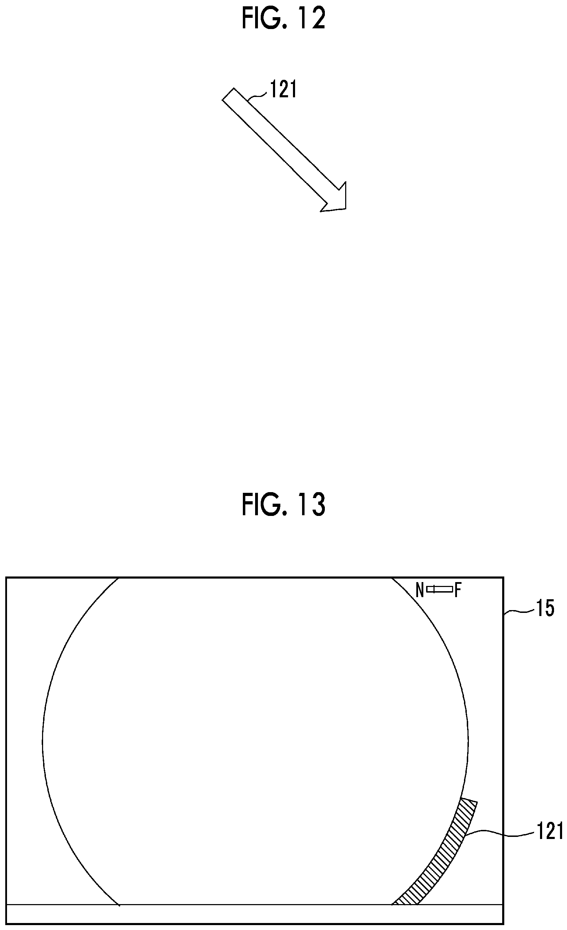

It is preferable that the direction display control unit 74 performs control for displaying the gravity direction indication with at least one of a color, a symbol, or a figure. As shown in Fig. 12, for example, a superimposition image in which a gravity direction indication 121 is superimposed on the normal image 81 in a shape of an arrow figure is generated, and the superimposition image is displayed on the display 15. It is preferable that the gravity direction indication 121 having an arrow shape is displayed such that a user can be reliably notified of the gravity direction 101 and the gravity direction indication does not interfere with the user's observation. Therefore, the arrow may be shown in a shape of a transparent figure.

In some cases, it is preferable that the direction display control unit 74 performs control for displaying the gravity direction indication 121 with a color around a region in which the normal image 81 is displayed. As shown in Fig. 13, for example, the gravity direction indication 121 is set as a frame of a specific color around a region in which the normal image 81 is displayed, and a superimposition image is generated by superimposing the gravity direction on the normal image 81, and the superimposition image is displayed on the display 15. It is preferable that the gravity direction indication 121 that is a frame of a specific color is displayed such that a user is reliably notified of the gravity direction and the gravity direction indication does not interfere with the user's observation. Therefore, the gravity direction indication may be shown by a method such as making a frame of a specific color around the normal image 81 a conspicuous color or blinking.

In a case where the direction display control unit 74 displays the gravity direction indication 121 in a form of an image on the display 15 in order to provide a notification of a detected gravity direction, the direction display control unit 74 performs control for aligning the gravity direction indication with the normal image 81 and then generating a superimposition image 122 by superimposing the gravity direction indication on the normal image 81. The superimposition image 122 generated by the direction display control unit 74 is displayed on the display 15.

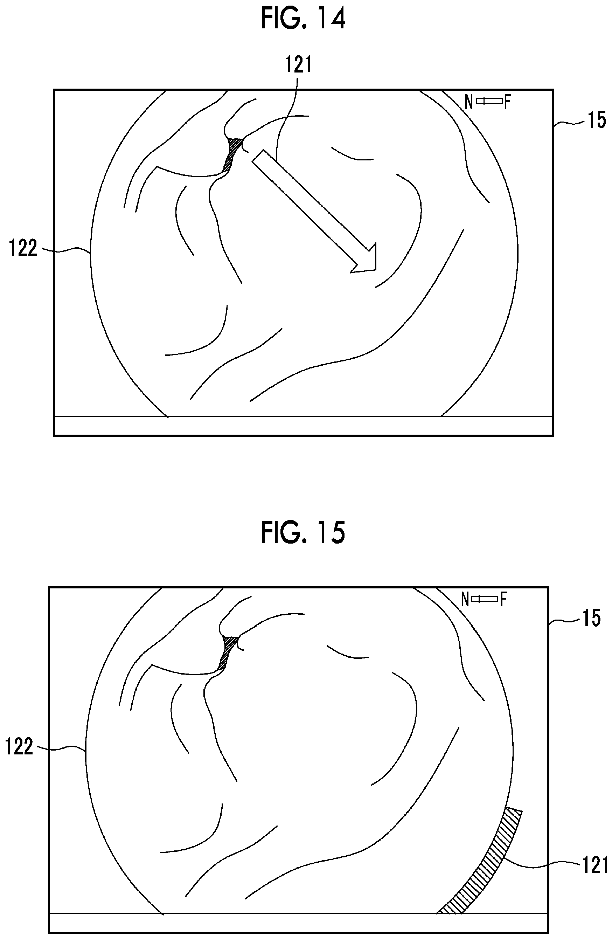

As shown in Fig. 14, the superimposition image 122 generated by the direction display control unit 74 is an image in which the gravity direction indication 121 shown in a shape of a transparent arrow figure is superimposed on the normal image 81. As shown in Fig. 15, the superimposition image 122 is an image in which a gravity direction indication 121 having a frame of a specific color around a region in which the normal image 81 is displayed is superimposed on the normal image 81. It is preferable that the superimposition image 122 is generated every time the normal image 81 is captured. Consequently, the superimposition image 122 can be displayed in real time while the observation target is imaged by the endoscope.

The display of the superimposition image 122, that is, the display of the gravity direction indication 121 can be turned on and off as appropriate. Therefore, in a case where it is considered that the gravity direction indication 121 hinders the progress of the endoscopy because the gravity direction indication 121 may interfere with the endoscopy, or the gravity direction indication 121 changes frequently and is troublesome, the normal image 81 may be displayed while the superimposition of the gravity direction indication 121 is turned off.

As described above, the medical image processing device 17 detects the gravity direction 101 in the normal image 81 on the basis of the normal image 81, generates the gravity direction indication 121 indicating the detected gravity direction 101, and displays the gravity direction indication on the display 15. Since the gravity direction 101 is detected on the basis of the normal image 81, the gravity direction 101 can be displayed without installing a special device for detecting the gravity direction in the endoscope, and thus the present invention is applicable to any endoscope regardless of the type of endoscope. In a case where the gravity direction 101 is detected through machine learning, it is possible to detect the gravity direction 101 with high accuracy by using a three-dimensional vector or the like. Since a display frequency, a display form, or the like of the gravity direction indication 121 can be set in advance by a user, display thereof can be set as desired by the user.

In a case where the gravity direction is detected on the basis of the normal image 81, it is preferable that the normal image 81 is obtained by imaging a subject including a liquid accumulation. The liquid is a cleaning liquid, blood, or other liquids. In a case where the direction detection unit 73 detects the gravity direction 101 for the normal image 81 obtained by imaging a subject including a liquid accumulation, the liquid accumulation can be detected by using an image processing technique. The liquid is collected and accumulated in the gravity direction 101. Therefore, a direction in which the liquid accumulation is detected in the normal image 81 can be the gravity direction 101. In a case where the machine learning technique is used, the gravity direction 101 can be detected more reliably and accurately. As described above, by detecting the gravity direction 101 on the basis of the normal image 81 in which the subject including the liquid accumulation is captured, the gravity direction 101 can be detected more reliably.

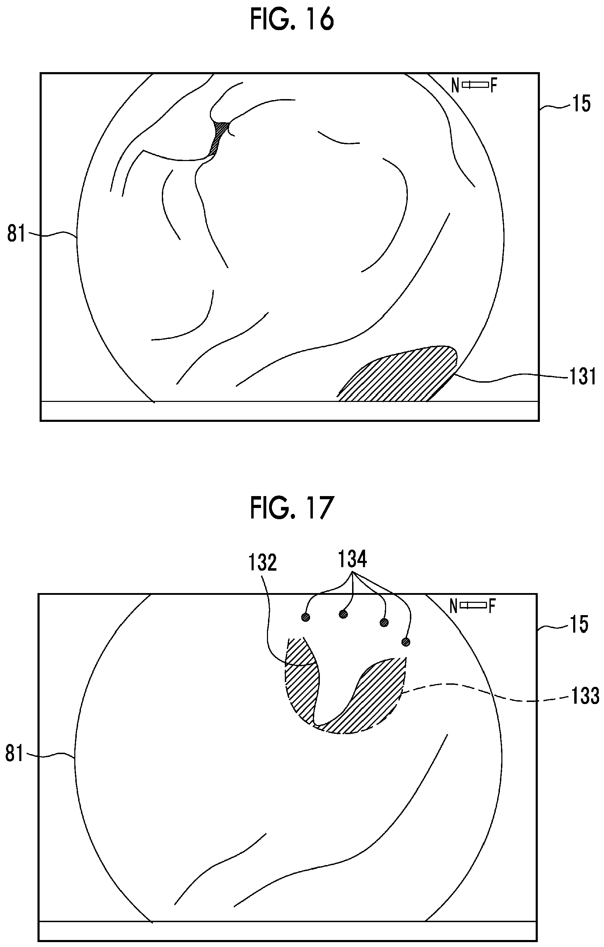

As shown in Fig. 16, the normal image 81 in which a subject includes a liquid accumulation 131 is the normal image 81 in which the liquid accumulation 131 is captured in any region of the normal image 81. As long as there is a liquid accumulation, a region in the normal image 81 is not limited. However, it is preferable to distinguish and recognize the liquid accumulation 131 present in the observation target and, for example, water droplets attached to the lens of the endoscope.

In a case where the gravity direction 101 is detected on the basis of the normal image 81 in which the subject including the liquid accumulation 131 is imaged by using the machine learning technique, it is preferable to use a learning model having performed learning using a learning image in which the normal image 81 obtained by imaging the subject including the liquid accumulation 131 is associated with the gravity direction 101 in the normal image 81.

As for the normal image 81 obtained by imaging the subject including the liquid accumulation 131, the gravity direction 101 may be detected by using a plurality of, for example, at least two normal images 81 captured at different time points. This is because there is a possibility that the gravity direction 101 can be detected more accurately by knowing a change in the liquid accumulation 131.

In a case where the gravity direction 101 is detected on the basis of the normal image 81, it is preferable that the normal image 81 is obtained by imaging a subject including the mucous membrane of which at least a part is incised. A case where a subject including an incised mucous membrane is, for example, a case where the mucous membrane is incised by using a treatment tool, endoscopic submucosal dissection (ESD) is performed, or endoscopic mucosal resection (EMR) is performed. Since ESD is performed under an endoscope, ESD has the advantage of being minimally invasive, while a doctor who is an operator is required to perform ESD with limited information based on endoscopic images or the like.

For example, ESD for resecting a tumor is performed according to procedures such as marking around the tumor, local injection into the submucosal layer, incision of the mucous membrane, peeling of the submucosal layer, and hemostasis. In a case where the submucosal layer is incised and the mucous membrane is peeled off, for example, in a case where the mucous membrane desired to be peeled off is present near the upper part, the peeled mucous membrane hangs down, this makes it impossible to visually recognize the depth of the hanging mucous membrane, and thus a location to be incised in the latter half may become invisible. Therefore, in a case where the upper mucous membrane is peeled off, the gravity direction is indicated, so that the incision procedure can be devised in advance, and a field of view can be secured until the latter half of the incision.

The mucous membrane of which at least a part is incised is a mucous membrane including a portion incised by a treatment tool or the like, and the degree of incision or a incision region is not limited. For example, in ESD, the mucous membrane is first incised, the incision around the tumor is advanced, and finally the mucous membrane containing the tumor is peeled off. Therefore, the normal image obtained by imaging a subject including at least a partially incised mucous membrane is preferably the normal image 81 in which the stages of incision of the mucous membrane and peeling of the submucosal layer in the ESD procedure are captured.

In a case where the direction detection unit 73 detects the gravity direction 101 for the normal image 81 obtained by imaging a subject including at least a partially incised mucous membrane, image processing and machine learning techniques may be used. With these techniques, the gravity direction 101 can be detected by detecting a position of the incised mucous membrane or the like in the subject captured in the normal image 81. In a case where the machine learning technique is used, the gravity direction 101 can be detected more reliably and accurately on the basis of a position, a shape, or the like of the incised mucous membrane. As described above, it is possible to detect the gravity direction 101 can be detected more reliably and accurately by detecting the gravity direction 101 on the basis of the normal image 81 in which a subject includes at least a partially incised the mucous membrane.

As shown in Fig. 17, the normal image 81 in which a subject includes at least a partially incised mucous membrane is the normal image 81 in which at least a partially incised mucous membrane 132 is captured in any region of the normal image 81. The mucous membrane 132 is a mucous membrane incised through ESD and a doctor proceeds with the incision along a marker 134 marked prior to the incision. After the incision, a submucosal layer 133 is exposed, and the incised mucous membrane 132 hangs down in the gravity direction 101. A subject including the mucous membrane 132 of which at least a part is incised preferably has the mucous membrane 132 that hangs down in the gravity direction 101 regardless of a region in the normal image 81. Due to a shape of the mucous membrane 132, the mucous membrane 132 hangs down, and the gravity direction 101 can be recognized according to a direction of the mucous membrane 132 in the normal image 81.

In a case where the gravity direction 101 is detected on the basis of the normal image 81 obtained by imaging a subject including the mucous membrane 132 of which at least a part is incised by using the machine learning technique, it is preferable to use a learning model having performed learning using a learning image in which the normal image 81 obtained by imaging the subject including the incised mucous membrane 132 is associated with the gravity direction 101 in the normal image 81.

As for the normal image 81 obtained by imaging a subject including the mucous membrane of which at least a part is incised, the gravity direction 101 may be detected by using a plurality of, for example, at least two normal images 81 captured at different time points. This is because there is a possibility that the gravity direction 101 can be detected more accurately by knowing a change in a position, a shape, or the like of the incised mucous membrane.

In a case where the gravity direction 101 is detected on the basis of the normal image 81, it is preferable that the normal image 81 is obtained by imaging a subject including a released liquid. During endoscopy, for example, a cleaning liquid for cleaning mucus may be released, a staining solution may be applied, or a liquid may be released from the tip part 12d of the endoscope. Specifically, a liquid such as a cleaning liquid is released by using the forceps port 12h or an air supply/water supply port (not shown) provided at the tip part 12d of the endoscope. In a case where the released liquid contacts an observation target, the liquid then falls in the gravity direction 101. Therefore, the gravity direction 101 can be detected by the direction detection unit 73 for the normal image 81 obtained by imaging the subject including the released liquid.

In a case where the direction detection unit 73 detects a gravity direction for the normal image 81 obtained by imaging a subject including the released liquid, a direction, a trajectory, or the like of the liquid after being released can be detected by using image processing or machine learning techniques. In a case where the machine learning technique is used, the gravity direction 101 can be detected more reliably and accurately. As described above, by detecting the gravity direction 101 on the basis of the normal image 81 in which the subject including the released liquid is captured, the gravity direction 101 can be detected more reliably.

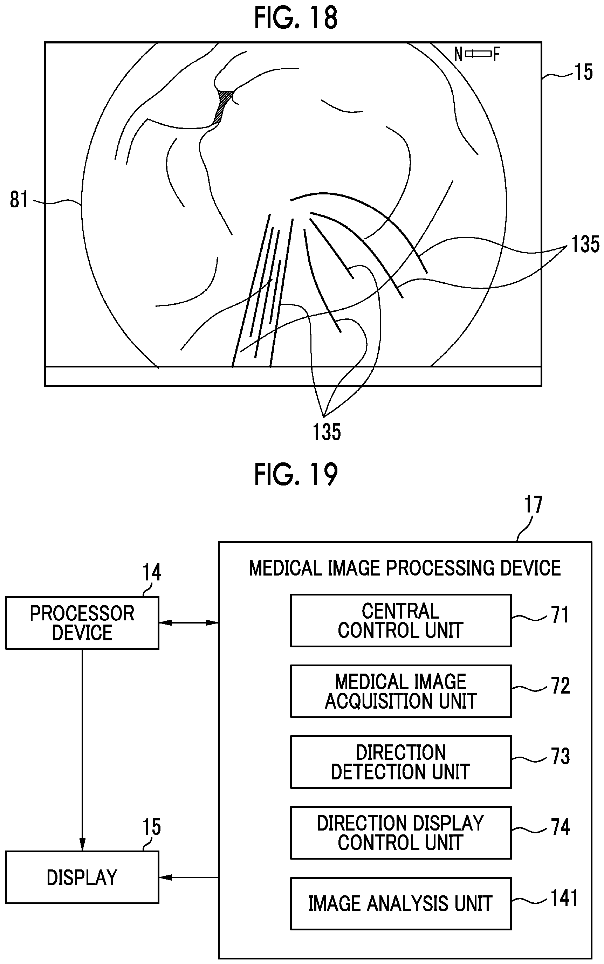

As shown in Fig. 18, the normal image 81 including a subject including a released liquid 135 is the normal image 81 in which the subject including the released liquid 135 is captured in any region of the normal image 81. As long as a subject includes the released liquid 135, a region in the normal image 81 is not limited, but the normal image 81 is an image in which a direction of the liquid 135 after the released liquid 135 contacts an observation target can be visually recognized. Therefore, the normal image 81 or the like in which a distance between the tip part 12d of the endoscope and the observation target is appropriate is preferable. According to the normal image 81 in which a direction of the liquid 135 can be visually recognized, it is possible to accurately detect the gravity direction 101.

In a case where the gravity direction 101 is detected on the basis of the normal image 81 obtained by imaging the subject including the released liquid 135 by using the machine learning technique, it is preferable to use a learning model having performed learning using a learning image in which the normal image 81 obtained by imaging the subject including the released liquid 135 is associated with the gravity direction 101 in the normal image 81.

As for the normal image 81 obtained by imaging the subject including the released liquid 135, the gravity direction 101 may be detected by using a plurality of, for example, at least two normal images 81 captured at different time points. This is because there is a possibility that the gravity direction 101 can be detected more accurately by knowing a change in a direction of the released liquid 135.

The direction display control unit 74 preferably controls whether or not to display the gravity direction indication 121. Whether or not to display the gravity direction indication 121 may be controlled on the basis of a user's instruction or the normal image 81.

The control of whether or not to display the gravity direction indication 121 on the basis of the user's instruction may be performed by using a user interface such as the keyboard 16, a touch panel of the display 15, a foot switch (not shown), or a device performing voice recognition. In a case of performing voice recognition, control for switching between display and non-display of the gravity direction indication 121 may be performed by a so-called wake word such as "gravity" and a voice instruction such as "display" or "stop".

In a case of controlling whether or not to display the gravity direction indication 121 on the basis of the normal image 81, the display is controlled according to a subject captured in the normal image 81, an imaging environment, a temporal change, or the like.

As shown in Fig. 19, the medical image processing device 17 includes an image analysis unit 141. The image analysis unit 141 analyzes a subject captured in the normal image 81, an imaging environment, or a temporal change. For example, the subject captured in the normal image 81 is a landmark, blood, a lesion or a state of the lesion, or a treatment tool. The state of the lesion is, for example, a state in which a subject is a mucous membrane marked in ESD or a mucous membrane raised by local injection, or a state in which the submucosal layer 133 is captured and the mucous membrane is peeled off. The imaging environment of the normal image 81 is a distance between the tip part 12d of the endoscope and an observation target that is a subject, the type of spectrum of illumination light, a zoom magnification, or the like. The temporal change of the normal image 81 is based on whether the temporal change is large or small. For example, in the normal image 81 captured within a certain period, in a case where a subject captured in the normal image 81 is different in the entire image, it may be determined that a temporal change is large and the endoscope is being moved, and in a case where a subject captured in the normal image 81 is substantially the same, it may be determined that a temporal change is small and the observation is performed without moving the endoscope.

The image analysis unit 141 performs analysis on the basis of the normal image 81, and ascertains the above-described details. In a case where it is determined that it is advantageous for a user to display the gravity direction indication 121 on the display 15 on the basis of the ascertained details, the direction display control unit 74 is instructed to display the gravity direction indication 121. On the other hand, in a case where it is determined that it is disadvantageous for the user to display the gravity direction indication 121 on the display 15 on the basis of the ascertained details, the direction display control unit 74 is instructed not to display the gravity direction indication 121. In this case, specifically, in a case where the gravity direction indication 121 is displayed, an instruction for stopping the display is given. The direction display control unit 74 controls whether or not to display the gravity direction indication 121 on the basis of the instruction from the image analysis unit 141.

The image analysis unit 141 performs analysis on the basis of the normal image 81 and controls whether or not to display the gravity direction indication 121, and thus the display of the gravity direction indication 121 is automatically turned on and off. For example, in a case where it is ascertained through analysis by the image analysis unit 141 that bleeding occurs in the observation target and thus bleeding occurs in the subject in the normal image 81, the direction display control unit 74 performs control for not displaying the gravity direction indication 121. In a case where it is ascertained through the analysis that the observation target has stopped bleeding and the bleeding has disappeared from the subject in the normal image 81, the direction display control unit 74 performs control for displaying the gravity direction indication 121.

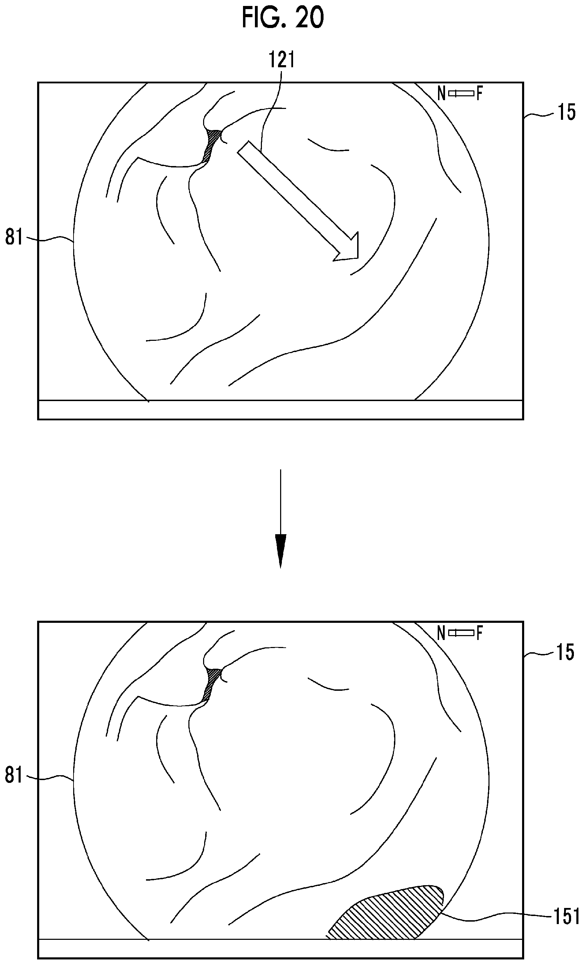

As shown in Fig. 20, the gravity direction indication 121 is displayed in the normal image 81 during a user's observation. The gravity direction indication 121 has a transparent arrow shape. During observation, bleeding occurs, blood 151 is included in the normal image 81. In this case, since the image analysis unit 141 performs analysis on the basis of the normal image 81 and detects the blood 151, the direction display control unit 74 performs control for not displaying the gravity direction indication 121. As a result, in the normal image 81 in which blood 151 is detected, the gravity direction indication 121 automatically disappears, and in a case where blood 151 is detected in the normal image 81, the gravity direction indication 121 is not still displayed. As described above, since the gravity direction indication 121 is not automatically displayed, the user can concentrate on hemostasis in a state in which the gravity direction indication 121 is not displayed on the normal image 81.

For example, in a case where it is ascertained through the analysis by the image analysis unit 141 that incision of the mucous membrane in ESD has been started, the direction display control unit 74 performs control for displaying the gravity direction indication 121. In a case where it is ascertained through the analysis that the incision in the ESD has been completed, the direction display control unit 74 performs control for not displaying the gravity direction indication 121.

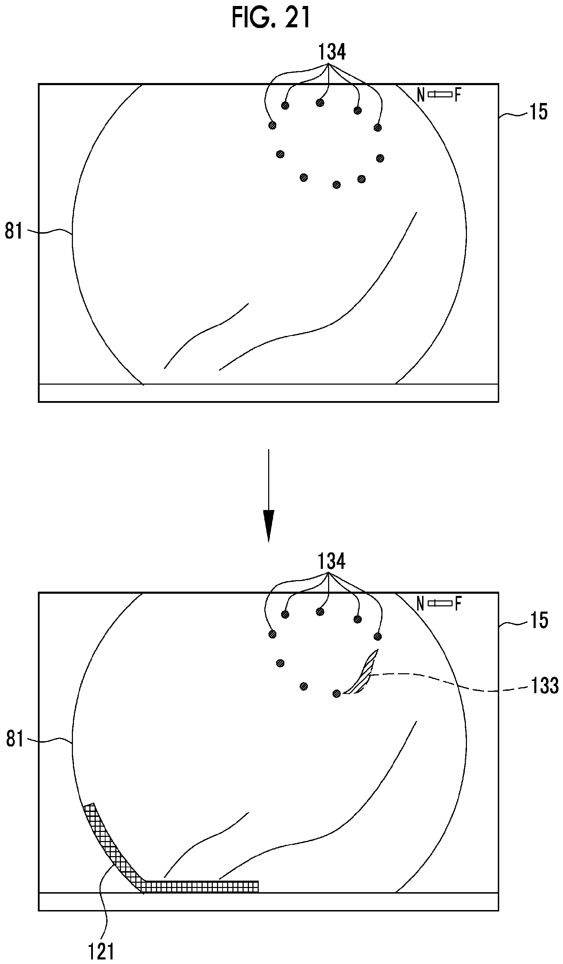

As shown in Fig. 21, the user performs ESD without displaying the gravity direction indication 121 in the normal image 81. In order to avoid the complexity of the figure, the reference numerals are given to only some portions. After the ESD progresses and markers 134 are applied to the mucous membrane through marking, incision is started. In a case where the incision is started, the image analysis unit 141 performs analysis on the basis of the normal image 81 and detects the incision of the mucous membrane, and thus the direction display control unit 74 performs control for displaying the gravity direction indication 121. The gravity direction indication 121 is a frame of a specific color around a preset region in which the normal image 81 is displayed. The gravity direction indication 121 is automatically displayed in a case where incision of the mucous membrane is detected. As described above, since the gravity direction indication 121 is automatically displayed, the user can proceed with ESD while ascertaining a direction in which the mucous membrane hangs down due to ESD in a state in which the gravity direction indication 121 is displayed on the normal image 81.

As described above, the image analysis unit 141 performs analysis on the basis of the normal image 81 and controls whether or not to display the gravity direction indication 121, and thus the user can ascertain the gravity direction 101 while concentrating on an operation of the endoscope, which is thus preferable.

A display form of the gravity direction indication 121 may be determined on the basis of the normal image 81. The display form of the gravity direction indication 121 is a form of the gravity direction indication 121, a position of the gravity direction indication 121 in the normal image 81, or the like. The form of the gravity direction indication 121 is a form of how the gravity direction indication 121 is displayed, and includes, for example, a color, a symbol, a figure, or a combination thereof. A display form such as blinking is also included.

The image analysis unit 141 performs analysis on the basis of the normal image 81. Details of the analysis and the like are the same as the analysis for controlling whether or not to display the gravity direction indication 121 described above. A display form of the gravity direction indication 121 is determined on the basis of details ascertained through the analysis, and information regarding the determined display form is sent to the direction display control unit 74. Regarding a display position of the gravity direction indication 121, a method of determining a display form in real time may be employed, such as computing an appropriate position in the normal image 81 and then appropriately displaying the gravity direction indication 121 at that position.

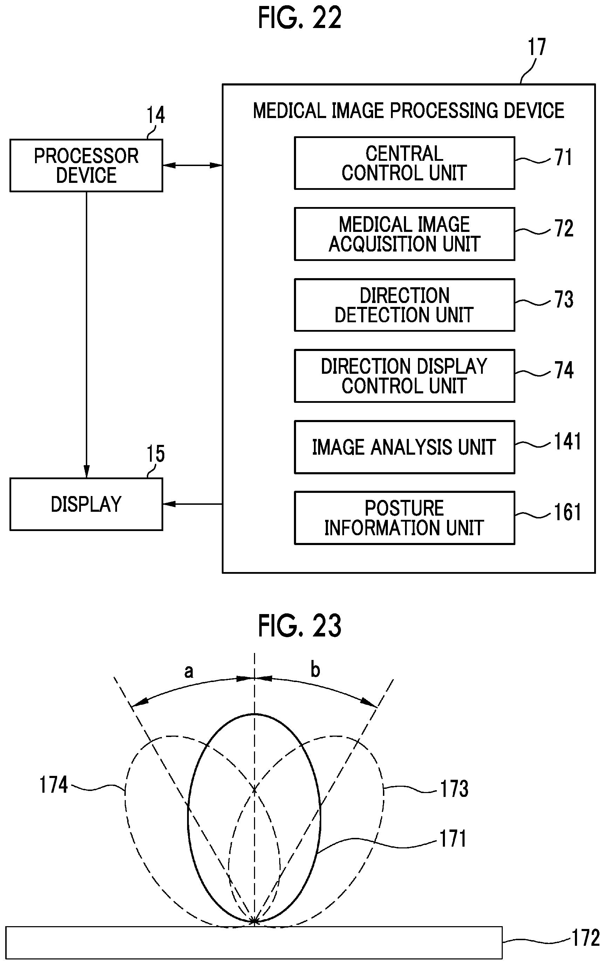

As shown in Fig. 22, the medical image processing device 17 may include a posture information unit 161 that records information regarding a patient's posture who is a target of endoscopy. The posture information unit 161 receives and records information regarding a patient's posture from a function of ascertaining a shape of the endoscope, a camera installed in an endoscopy examination room and imaging a patient's posture, or the like. The patient's posture is a position of the patient's body in a case where the patient is undergoing endoscopy. The information regarding the patient's posture is, for example, information regarding the patient's posture such as how much a reference posture is tilted back and forth from a reference with a longitudinal direction of the lying patient's body as an axis.

As shown in Fig. 23, the posture information unit 161 ascertains a position of a patient 171 in a reference posture on a bed 172 by using a camera (not shown) installed in a direction of the patient's head, and receives information regarding the patient's posture, and records the information with time data. Fig. 23 schematically shows a patient in a case where an image is captured by a camera installed in a direction of the patient's head, and a patient's posture will be described. For example, information regarding a patient's posture is information regarding a patient 174 tilted backward at an angle a from the reference position at a certain time, and information regarding a patient 173 tilted forward at an angle b from the reference position at another time. In a case of referring to a tilt of the patient, the front is the front as seen from the patient, and the back is the back as seen from the patient.

By using the information regarding the patient's posture recorded in the posture information unit 161 to obtain the patient's posture in more detail, the precision and accuracy of the gravity direction 101 detected by the direction detection unit 73 can be further improved.

In the above embodiment, the present invention is applied to a case of processing an endoscopic image, but is also applicable to a processor device, a medical image processing device, a medical image processing system, or the like processing a medical image other than an endoscopic image.

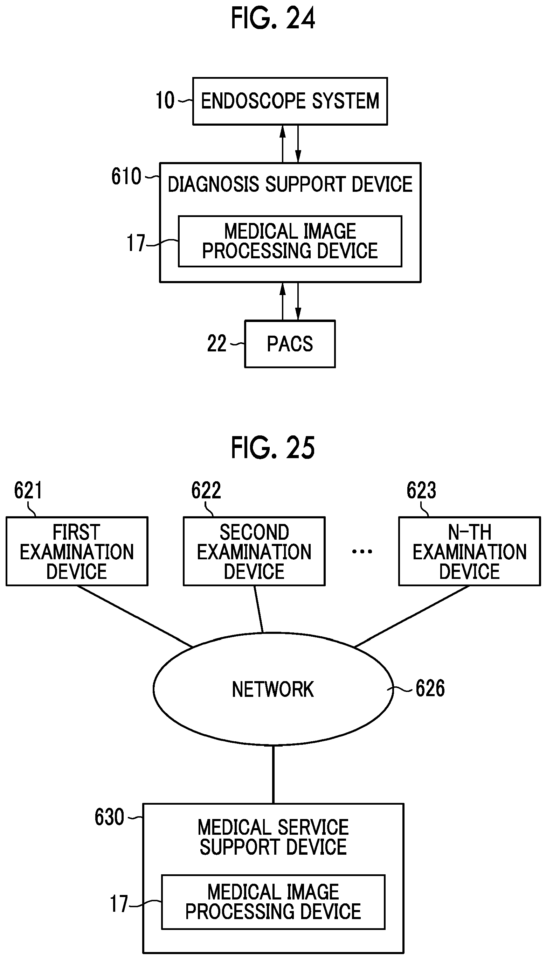

As shown in Fig. 24, a part or the whole of the image processing unit 55 and/or the central control unit 58 of the endoscope system 10 may be provided in a diagnosis support device 610 that acquires an image picked up by the endoscope 12 directly from the endoscope system 10 or indirectly from a picture archiving and communication systems (PACS) 22. Similarly, a part or the whole of the medical image processing device 17 of the endoscope system 10 may be provided in a diagnosis support device 610 that acquires an image picked up by the endoscope 12 directly from the endoscope system 10 or indirectly from a picture archiving and communication systems (PACS) 22.

As shown in Fig. 25, a part or the whole of the image processing unit 55 and/or the central control unit 58 or a part or the whole of the medical image processing device 17 of the endoscope system 10 may be provided in a medical service support device 630 including the endoscope system 10 and connected to various examination devices such as a first examination device 621, a second examination device 622, ..., and an N-th examination device 623 via a network 626.

In the above embodiment, hardware structures of processing units executing various processes, such as the light source processor, the central control unit 58, the image acquisition unit 51, the DSP 52, the noise reduction unit 53, the memory 54, the image processing unit 55, the display control unit 56, and the video signal generation unit 57 which are included in the processor device 14 including the first processor, and the central control unit 71, the medical image acquisition unit 72, the direction detection unit 73, the direction display control unit 74, the image analysis unit 141, and the posture information unit 161 that are included in the medical image processing device 17 including the second processor are various processors as described below. The various processors include a programmable logic device (PLD), which is a processor of which a circuit configuration can be changed after manufacturing, such as a central processing unit (CPU) or a field programmable gate array (FPGA) that is a general-purpose processor that executes software (programs) and functions as various processing units, a dedicated electric circuit that is a processor having a circuit configuration specially designed to execute various processes, and the like.

Includes Programmable Logic Device (PLD), which is a processor whose circuit configuration can be changed after manufacturing, and dedicated electric circuit, which is a processor with a circuit configuration specially designed to execute various processes.

One processing unit may be configured with one of these various processors, or may be configured with a combination of two or more processors of the same type or different types (for example, a plurality of FPGAs or a combination of a CPU and an FPGA). A plurality of processing units may be configured by one processor. As an example of configuring a plurality of processing units with one processor, first, there is a form in which one processor is configured by a combination of one or more CPUs and software, as typified by a computer used for a client or a server, and this processor functions as a plurality of processing units. Second, as typified by system on chip (SoC), there is a form in which a processor that realizes functions of the entire system including a plurality of processing units with one integrated circuit (IC) chip is used. As described above, the various processing units are configured by using one or more of the above various processors as a hardware structure.

The hardware structure of these various processors is, more specifically, an electric circuit (circuitry) in which circuit elements such as semiconductor elements are combined.

### Explanation of References

- 10:: endoscope system
- 12:: endoscope
- 12a:: insertion part
- 12b:: operating part
- 12c:: bendable part
- 12d:: tip part
- 12e:: angle knob
- 12f:: zoom operating part
- 12g:: mode selector switch
- 12h:: forceps port
- 13:: light source device
- 14:: processor device
- 15:: display
- 16:: keyboard
- 17:: medical image processing device
- 20:: light source unit
- 20a:: V-LED
- 20b:: B-LED
- 20c:: G-LED
- 20d:: R-LED
- 21:: light source processor
- 22:: PACS
- 30a:: Illumination optical system
- 30b:: image pick-up optical system
- 41:: light guide
- 42:: illumination lens
- 43:: objective lens
- 44:: zoom lens
- 45:: image pick-up sensor
- 46:: CDS/AGC circuit
- 47:: A/D converter
- 51:: image acquisition unit
- 52:: DSP
- 53:: noise reduction unit
- 54:: memory
- 55:: image processing unit
- 56:: display control unit
- 57:: video signal generation unit
- 58, 71:: central control unit
- 61:: normal image processing unit
- 62:: special image processing unit
- 72:: medical image acquisition unit
- 73:: direction detection unit
- 74:: direction display control unit
- 81:: normal image
- 91:: pyloric portion
- 92:: lesser curvature
- 93:: greater curvature
- 94:: anterior wall
- 95:: posterior wall
- 96:: vestibular portion
- 101:: gravity direction
- 111:: finger
- 112:: arrow
- 113:: gravity direction specification screen
- 113a:: rotatable indication
- 114:: three-dimensional schema diagram of stomach
- 115:: imaging position
- 116:: camera position
- 121:: gravity direction indication
- 122:: superimposition image
- 131:: liquid accumulation
- 132:: mucous membrane
- 133:: submucosal layer
- 134:: marker
- 135:: liquid
- 141:: image analysis unit
- 151:: blood
- 161:: posture information unit
- 171:: patient in reference posture
- 172:: bed
- 173:: patient tilted backward
- 174:: patient tilted forward
- 610:: diagnosis support device
- 621:: first examination device
- 622:: second examination device
- 623:: N-th examination device
- 626:: network
- 630:: medical service support device
- x, y:: direction
- a, b:: tilt

## Claims

1. A medical image processing device comprising a processor configured to:
acquire an endoscopic image obtained by imaging a subject with an endoscope;
detect a gravity direction in the endoscopic image on the basis of the endoscopic image;
generate a gravity direction indication indicating the gravity direction; and
perform control for displaying the gravity direction indication on a display.

2. The medical image processing device according to claim 1,
wherein the subject includes a liquid accumulation.

3. The medical image processing device according to claim 1 or 2, wherein
the subject includes a mucous membrane of which at least a part is incised.

4. The medical image processing device according to any one of claims 1 to 3,
wherein the subject includes a released liquid.

5. The medical image processing device according to any one of claims 1 to 4,
wherein the processor is configured to detect the gravity direction in the endoscopic image by using the endoscopic image and a learning model that outputs the gravity direction in the endoscopic image in a case where the endoscopic image is input.

6. The medical image processing device according to any one of claims 1 to 5,
wherein the processor is configured to:
generate a superimposition image in which the gravity direction indication is superimposed on the medical image; and
perform control for displaying the superimposition image on the display.

7. The medical image processing device according to any one of claims 1 to 6,
wherein the processor is configured to perform control for displaying the gravity direction indication with at least one of a color, a symbol, or a figure.

8. The medical image processing device according to any one of claims 1 to 7,
wherein the processor is configured to perform control for displaying the gravity direction indication with a color around the endoscopic image.

9. The medical image processing device according to any one of claims 1 to 8,
wherein the processor is configured to control whether or not to display the gravity direction indication on the basis of a user's instruction.

10. The medical image processing device according to any one of claims 1 to 8, wherein
the processor is configured to control whether or not to display the gravity direction indication on the basis of the endoscopic image.

11. The medical image processing device according to any one of claims 1 to 10,
wherein the processor is configured to determine a display mode of the gravity direction indication on the basis of the endoscopic image.

12. An operation method for a medical image processing device, comprising:
a step of acquiring an endoscopic image obtained by imaging a subject with an endoscope;
a step of detecting a gravity direction in the endoscopic image on the basis of the endoscopic image;
a step of generating a gravity direction indication indicating the gravity direction; and
a step of performing control for displaying the gravity direction indication on a display.

13. An endoscope system comprising:
the medical image processing device according to any one of claims 1 to 11; and
the endoscope.
